# EUROPEAN PATENT APPLICATION

(11) **EP 4 803 115 A1**
(43) Date of publication of application: **09.09.2026**
(21) Application number: 26162775.6
(22) Date of filing: 06.03.2026
(51) Int. Cl.: A61M 1/34, A61M 1/36, A61M 1/38

(54) **BLOOD PROCESSING SYSTEM, DEVICES, AND METHODS FOR OPTIMIZING FLUID TRANSFER AND CONFIRMATION OF AN EMPTY CONTAINER**

(30) Priority: 07.03.2025 US 202563768561 P
(71) Applicant: Fenwal, Inc., Lake Zurich, IL 60047 (US)
(72) Inventor: DICOLA, Nicholas R., Lake Zurich, 60047 (US); MAHER, Jeffrey R., Lake Zurich, 60047 (US); QUEZADA, Francesca S., Lake Zurich, 60047 (US)
(74) Representative: Maikowski & Ninnemann Patentanwälte Partnerschaft mbB

(57) **Abstract**

Blood processing systems, devices, and methods including a durable hardware component, a disposable fluid flow circuit, and a controller configured to determine an optimum flow rate. More particularly, blood processing systems and devices including a controller configured to determine an optimum flow rate for return fluid and to determine false positive empty container detections.

## Description

### Field of the Disclosure

The present disclosure is directed to systems, devices, and methods for blood processing procedures. More particularly, the present disclosure is directed to blood processing systems and devices configured to minimize the time a subject is connected to the blood processing system and/or device. Even more particularly, the present disclosure is directed to blood processing systems and devices configured to minimize the time required to reinfuse blood components and to detect false positive indications that reinfusion is complete.

### Background

Various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source. Removing only particular constituents is advantageous when the blood source is a human donor because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

Such blood processing systems typically include in-process containers and red blood cell (RBC) containers that are emptied and their contents returned to the donor during reinfusion. While most apheresis procedures conclude as planned, early termination may occur due to various factors including, but not limited to, donor request, vascular access issues, or hardware errors, necessitating the return of additional uncollected products beyond nominal amounts.

Return fluids to be reinfused to a patient often vary in their composition, particularly in the case of early procedure termination. This variability creates differing hematocrit (HCT) levels of return fluid, which directly affect fluid viscosity. The relationship between optimal flow rate and fluid viscosity is inverse, wherein higher viscosity fluids (corresponding to higher HCT) require lower flow rates, while lower viscosity fluids can accommodate higher flow rates.

Typically, blood processing devices do not determine, in real-time, the composition of return fluids and thus use predetermined flow rates for reinfusion. In some examples, return fluid is reintroduced to a patient at a predetermined flow rate such as, for example, 150 mL/min. Other systems, may use a slower fixed return flow rate of approximately 60 mL/min.

In some instances, such predetermined fixed rates are suboptimal, especially when HCT is low following non-standard processes such as air purges or plasma transfer states. For example, air purges may introduce saline, which may dramatically lower the HCT of the fluid to be transferred. Additionally, during plasma transfer, the HCT will vary depending on the level of completion of the plasma transfer. Thus, if the plasma transfer is completed, the HCT will be lower than if it was interrupted midway through.

Additionally, predetermined fixed flow rates can be suboptimal when the HCT is too high. For instance, occlusions may occur when flow rates exceed the capability of the source container to support fluid movement due to viscosity constraints. Because higher viscosity fluids do not flow as easily as lower viscosity fluids, the maximum flow rate that can be achieved with a higher viscosity fluid is lower than for lower viscosity fluids. Under such conditions, flexible components within the fluid pathway are susceptible to collapse, effectively creating a valve-like obstruction. Often, a pump attempts to move a certain volume with each pump stroke, and if the flow of fluid is insufficient to maintain that volume, a negative pressure can be created in the tubing, creating a risk that the tubing will collapse to support the flow rate. The flexible components, which may be particularly prone to collapse and thereby impeding fluid flow include, but are not limited to, air traps and separation systems, separation chambers, mixing chambers, and flexible tubing.

When such collapses occur, current systems may erroneously interpret static weight readings as indicating an empty container, thereby leading to premature termination of reinfusion procedures. Such "false positive" indications cause disruptions to blood processing procedures, thus resulting in suboptimal procedure times and extending donor needle-in times. In some instances, a false positive indication may not be detected until an operator checks the system. Additionally, multiple false positive indications can occur during a single blood processing procedure.

Thus, there is a need for improved systems, devices, and methods for reinfusing a return fluid during a blood processing procedure.

### Summary

In one aspect, a blood processing system is provided. The blood processing system includes a reusable separation device, wherein the separation device includes a separator and a weight scale. The blood processing system also includes a disposable fluid circuit including a separation chamber and a fluid container. The disposable fluid circuit is configured to be associated with the reusable separation device and the fluid container is configured to be associated with the weight scale. The reusable separation device includes a controller configured to execute a blood processing procedure and to determine an optimum flow rate for flowing a fluid from the fluid container.

In another aspect, a blood processing device is provided. The blood processing device includes a separator configured to be associated with a separation chamber of a disposable fluid circuit, a plurality of pumps configured to pump fluids throughout the disposable fluid circuit, a weight scale configured to be associated with a fluid container of the disposable fluid circuit, and a controller configured to execute a blood processing procedure and to determine an optimum flow rate for flowing a fluid from the fluid container.

In yet another aspect, a method for processing blood is provided. The method includes separating whole blood in a blood processing system configured to execute a blood processing procedure. The system includes a reusable separation device, wherein the separation device includes a separator and a weight scale and a disposable fluid circuit. The disposable fluid circuit includes a separation chamber and a fluid container. The disposable fluid circuit is configured to be associated with the reusable separation device and the fluid container is configured to be associated with the weight scale. The reusable separation device also includes a controller configured to execute the blood processing procedure. The method further includes determining an optimum flow rate for flowing a fluid from the fluid container.

### Brief Description of the Drawings

FIG. 1 is a perspective view of an exemplary fluid processing device.
FIG. 2 is a schematic view of an exemplary disposable fluid flow circuit that may be mounted to the fluid processing device of FIG. 1 to complete a fluid processing system according to an aspect of the present disclosure.
FIG. 3 is a top plan view of an exemplary cassette of the fluid flow circuit of FIG. 2, which can be actuated to perform a variety of different fluid processing procedures in association with the fluid processing device shown in FIG. 1.
FIG. 4 is a schematic view of the fluid flow circuit of FIG. 2 mounted on the fluid processing device of FIG. 1, showing the system carrying out a fluid processing procedure.
FIG. 5 is a flowchart showing an example of a method of determining the optimum flow rate to flow a fluid from a fluid container during a blood processing procedure.
FIG. 6 is a flowchart showing another example of a method of determining the optimum flow rate to flow a fluid from a fluid container during a blood processing procedure.
FIG. 7 is a flowchart showing a method of determining a false positive empty detection during a blood processing procedure.
FIG. 8 is a flowchart showing another method of determining a false positive empty detection during a blood processing procedure.
FIG. 9 is a flowchart showing another method of determining a false positive empty detection during a blood processing procedure.

### Detailed Description of the Embodiments

A more detailed description of the systems and methods in accordance with the present disclosure is set forth below. It will be understood that the description below of specific devices and methods is intended to be exemplary, and not exhaustive of all possible variations or applications.

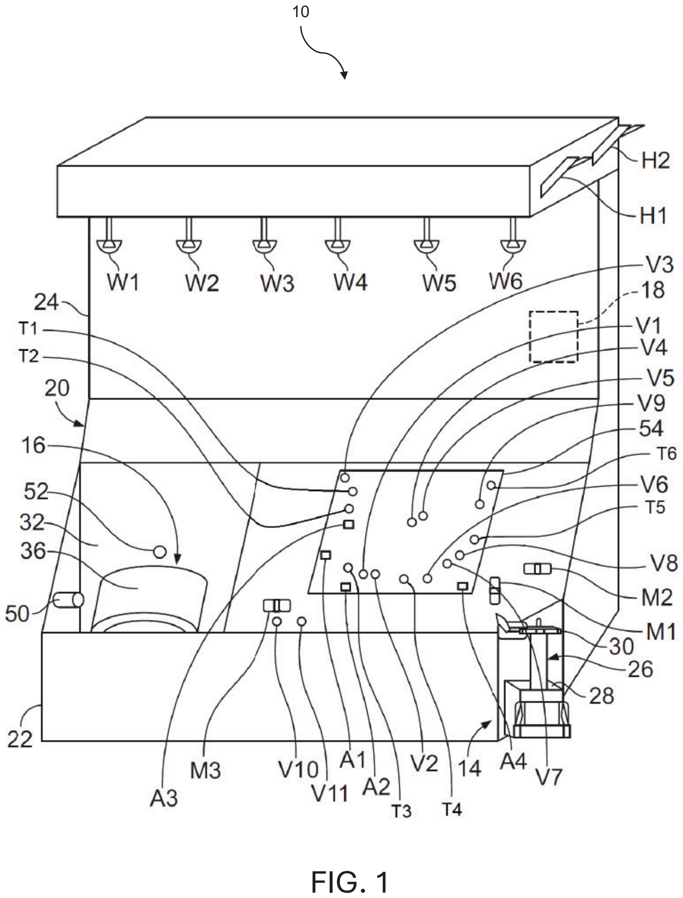

FIGS. 1-4 show components of a fluid processing system that embody various aspects of the present subject matter. Use of the system for separating blood into two or more components and collecting at least one of the components will be described herein, though it should be understood that systems according to the present disclosure can be used for processing a variety of fluids, which may include bodily fluids and non-bodily fluids.

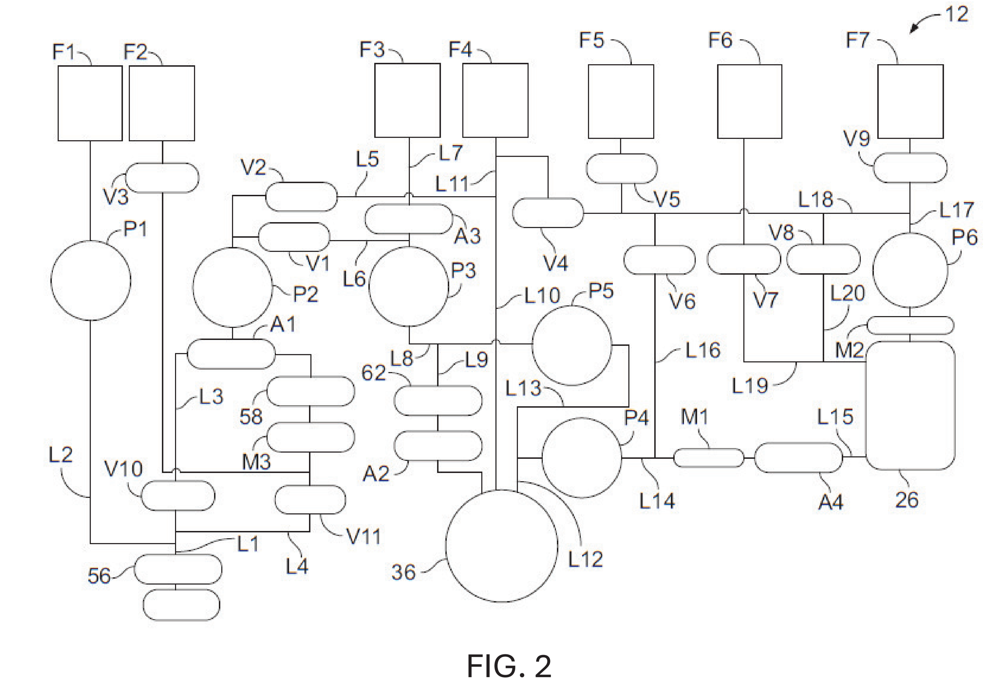

In one embodiment, the system includes two principal components, a durable and reusable fluid processing device 10 (FIG. 1) and a disposable fluid flow circuit 12 (schematically shown in FIG. 2). The illustrated fluid processing device 10 includes a spinning membrane separator drive unit 14, a centrifuge or centrifugal separator 16, additional components that control fluid flow through the disposable flow circuit 12, and a controller 18, which governs the operation of the other components of the fluid processing device 10 to perform a fluid processing procedure. A fluid processing device 10 of the type shown in FIG. 1 is described in greater detail in PCT Patent Application Publication No. WO 2018/053217 A1, which is hereby incorporated herein by reference. While the principles described herein may be employed when using the fluid processing device 10 of FIG. 1, it should be understood that these same principles may be applied to other fluid processing devices, including devices employing single separation technologies or approaches.

### I. The Durable Fluid Processing Device

The fluid processing device 10 (FIG. 1) is configured as a durable item that is capable of long-term use. It should be understood that the fluid processing device 10 of FIG. 1 is merely exemplary of one possible configuration and that fluid processing devices according to the present disclosure may be differently configured.

In the illustrated embodiment, the fluid processing device 10 is embodied in a single housing or case 20. The illustrated case 20 includes a generally horizontal portion 22 (which may include an inclined or angled face or upper surface for enhanced visibility and ergonomics) and a generally vertical portion 24. The spinning membrane separator drive unit 14 and the centrifugal separator 16 are shown as being incorporated into the generally horizontal portion 22 of the case 20, while the controller 18 is shown as being incorporated into the generally vertical portion 24.

### A. Spinning Membrane Separator Drive Unit

The fluid processing device 10 includes a spinner support or spinning membrane separator drive unit 14 for accommodating a generally cylindrical spinning membrane separator 26 of the fluid flow circuit 12. U.S. Patent No. 5,194,145 (which is hereby incorporated herein by reference) describes an exemplary spinning membrane separator drive unit that would be suitable for incorporation into the fluid processing device 10, but it should be understood that the spinning membrane separator drive unit 14 may be differently configured without departing from the scope of the present disclosure.

The illustrated spinning membrane separator drive unit 14 has a base 28 configured to receive a lower portion of the spinning membrane separator 26 and an upper end cap 30 to receive an upper portion of the spinning membrane separator 26. Preferably, the upper end cap 30 is positioned directly above the base 28 to orient a spinning membrane separator 26 received by the spinning membrane separator drive unit 14 vertically and to define a vertical axis about which the spinning membrane separator 26 is spun. While it may be advantageous for the spinning membrane separator drive unit 14 to vertically orient a spinning membrane separator 26, it is also within the scope of the present disclosure for the spinning membrane separator 26 to be differently oriented when mounted to the fluid processing device 10.

In one embodiment, one of the base 28 and upper end cap 30 of the spinning membrane separator drive unit 14 is movable with respect to the other, which may allow differently sized spinning membrane separators 26 to be received by the spinning membrane separator drive unit 14. For example, the upper end cap 30 may be translated vertically with respect to the base 28 and locked in a plurality of different positions, with each locking position corresponding to a differently sized spinning membrane separator 26.

At least one of the base 28 and the upper end cap 30 is configured to spin one or more components of the spinning membrane separator 26 about the axis defined by the spinning membrane separator drive unit 14. The mechanism by which the spinning membrane separator drive unit 14 spins one or more components of the spinning membrane separator 26 may vary without departing from the scope of the present disclosure. In one embodiment, a component of the spinning membrane separator 26 to be spun includes at least one element configured to be acted upon by a magnet (e.g., a metallic material), while the spinning membrane separator drive unit 14 includes a magnet (e.g., a series of magnetic coils or semi-circular arcs). By modulating the magnetic field acting upon the aforementioned element of the spinning membrane separator 26, the component or components of the spinning membrane separator 26 may be made to spin in different directions and at varying speeds. In other embodiments, different mechanisms may be employed to spin the component or components of the spinning membrane separator 26.

Regardless of the mechanism by which the spinning membrane separator drive unit 14 spins the component or components of the spinning membrane separator 26, the component or components of the spinning membrane separator 26 is/are preferably spun at a speed that is sufficient to create Taylor vortices in a gap between the spinning component and a stationary component of the spinning membrane separator 26 (or a component that spins at a different speed). Fluid to be separated within the spinning membrane separator 26 flows through this gap, and filtration may be dramatically improved by the creation of Taylor vortices.

### B. Centrifugal Separator

As for the centrifugal separator 16, it includes a centrifuge compartment 32 that receives a centrifugal separation chamber 36 of the fluid flow circuit 12, as well as other components of the centrifugal separator 16. Further details as to the configuration and operation of an exemplary centrifugal separator are set forth in PCT Patent Application Publication No. WO 2018/053217 A1.

In the embodiment of FIGS. 1-2, fluid (e.g., anticoagulated whole blood) is introduced into the centrifugal separation chamber 36 by an umbilicus, with the fluid being separated into a layer of less dense components (e.g., platelet-rich plasma, in the case of blood being separated) and a layer of more dense components (e.g., packed red blood cells) within the centrifugal separation chamber 36 as a result of centrifugal forces as it rotates. Components of an interface monitoring system may be positioned within the centrifuge compartment 32 to oversee separation of fluid within the centrifugal separation chamber 36. The interface monitoring system may include a light source 50 and a light detector 52, which is positioned and oriented to receive at least a portion of the light emitted by the light source 50.

The orientation of the various components of the interface monitoring system depends at least in part on the particular configuration of the centrifugal separation chamber 36. In general, though, the light source 50 emits a light beam (e.g., a laser light beam) through the separated fluid components within the centrifugal separation chamber 36 (which may be formed of a material that substantially transmits the light or at least a particular wavelength of the light without absorbing it). A portion of the light reaches the light detector 52, which transmits a signal to the controller 18 that is indicative of the location of an interface between the separated fluid components. If the controller 18 determines that the interface is in the wrong location (which can affect the separation efficiency of the centrifugal separator 16 and/or the quality of the separated fluid components), then it can issue commands to the appropriate components of the fluid processing device 10 to modify their operation so as to move the interface to the proper location.

### C. Other Components Of The Fluid Processing Device

In addition to the spinning membrane separator drive unit 14 and the centrifugal separator 16, the fluid processing device 10 may include other components compactly arranged to aid fluid processing.

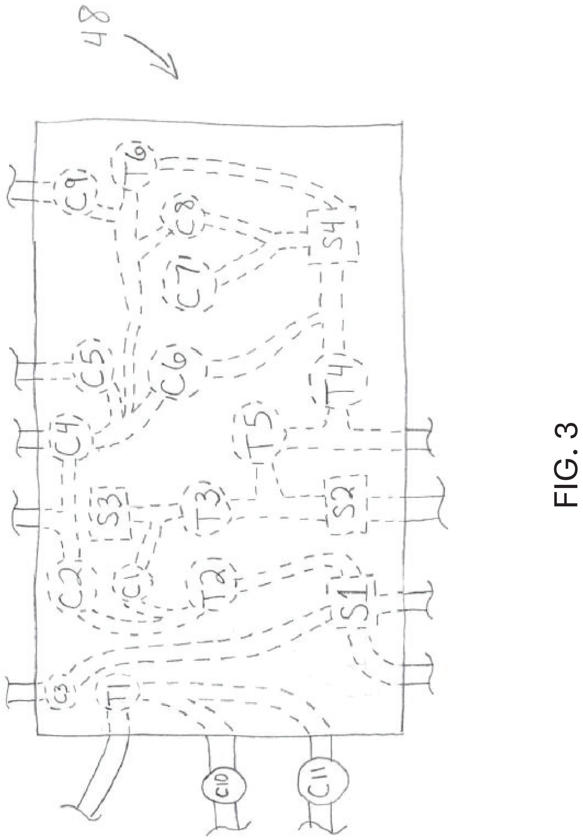

The generally horizontal portion 22 of the case 20 of the illustrated fluid processing device 10 includes a cassette station 54, which accommodates a flow control cassette 48 of the fluid flow circuit 12. An exemplary flow control cassette 48 (which will be described in greater detail herein) is shown in FIG. 3. In one embodiment, the cassette station 54 is similarly configured to the pneumatic-actuated pump and valve station described in U.S. Patent Application Publication No. 2006/0161092. The illustrated cassette station 54 includes a plurality of clamps or valves V1-V9 (FIG. 1), which move between a plurality of positions (e.g., between a retracted or lowered position and an actuated or raised position) to selectively contact or otherwise interact with corresponding valve stations C1-C9 of the flow control cassette 48 of the fluid flow circuit 12 (FIG. 3). Depending on the configuration of the fluid flow circuit 12, its cassette 48 may not include a valve station C1-C9 for each valve V1-V9 of the cassette station 54, in which case fewer than all of the valves V1-V9 will be used in a fluid processing procedure.

In the actuated position, a valve V1-V9 engages the associated valve station C1-C9 to prevent fluid flow through that valve station C1-C9 (e.g., by closing one or more ports associated with the valve station C1-C9, thereby preventing fluid flow through that port or ports). In the retracted position, a valve V1-V9 is disengaged from the associated valve station C1-C9 (or less forcefully contacts the associated valve station C1-C9 than when in the actuated position) to allow fluid flow through that valve station C1-C9 (e.g., by opening one or more ports associated with the valve station C1-C9, thereby allowing fluid flow through that port or ports). Additional clamps or valves V10 and V11 may be positioned outside of the cassette station 54 to interact with portions of valve stations C10 and C11 (which may be lengths of tubing) of the fluid flow circuit 12 to selectively allow and prevent fluid flow therethrough. The valves V1-V9 and corresponding valve stations C1-C9 of the cassette station 54 and cassette 48 may be differently configured and operate differently from the valves V10 and V11 and the valve stations C10 and C11 that are spaced away from the cassette station 54.

The cassette station 54 may be provided with additional components, such as pressure sensors A1-A4, which interact with sensor stations S1-S4 of the cassette 48 to monitor the pressure at various locations of the fluid flow circuit 12. For example, if the fluid source is a human subject, one or more of the pressure sensors A1-A4 may be configured to monitor the pressure of the subject's vein during blood draw and return. Other pressure sensors A1-A4 may monitor the pressure of the spinning membrane separator 26 and the centrifugal separation chamber 36. The controller 18 may receive signals from the pressure sensors A1-A4 that are indicative of the pressure within the fluid flow circuit 12 and, if a signal indicates a low- or highpressure condition, the controller 18 may initiate an alarm or error condition to alert an operator to the condition and/or to attempt to bring the pressure to an acceptable level without operator intervention.

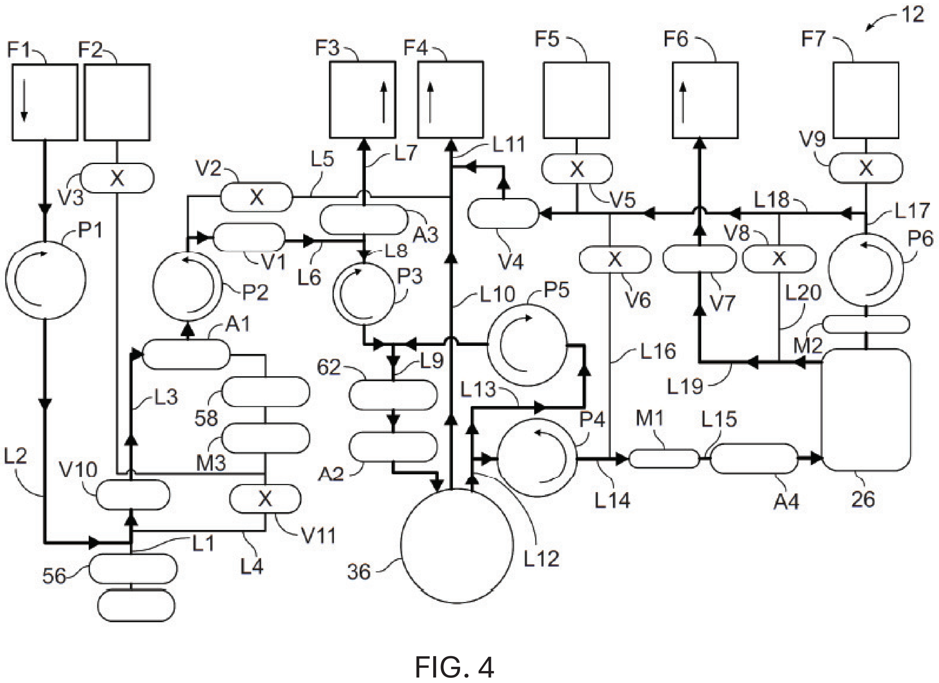

The fluid processing device 10 may also include a plurality of pumps P1-P6 (which may be collectively referred to as a pump assembly or pump system) to cause fluid to flow through the fluid flow circuit 12. The pumps P1-P6 may be differently or similarly configured and/or function similarly or differently from each other. For example, the pumps P1-P6 could be configured as either peristaltic or pneumatic pumps. In a particular example, the pumps P1-P6 are configured as pneumatic pumps, which may be generally configured as described in U.S. Patent Application Publication No. 2006/0161092. An actuator of each pump P1-P6 is controlled by the controller 18 to alternately apply positive and negative pressures to a flexible membrane or diaphragm of a different pump chamber T1-T6 defined by the flow control cassette 48 (FIG. 4) so as to cause fluid to flow through a portion of the fluid flow circuit 12. The configurations and operation of the pump system and the controller 18 will be described in greater detail herein.

The illustrated fluid processing device 10 also includes a spinner inlet sensor M1 (FIG. 1) for determining one or more properties of a fluid flowing into a spinning membrane separator 26 mounted within the spinning membrane separator drive unit 14. If the fluid flowing into the spinning membrane separator 26 is whole blood (which may include anticoagulated whole blood), the spinner inlet sensor M1 may be configured to determine the hematocrit of the blood flowing into the spinning membrane separator 26. If the fluid flowing into the spinning membrane separator 26 is platelet-rich plasma, the spinner inlet sensor M1 may be configured to determine the platelet concentration of platelet-rich plasma flowing into the spinning membrane separator 26. The spinner inlet sensor M1 may detect the one or more properties of a fluid by optically monitoring the fluid as it flows through tubing of the fluid flow circuit 12, or by any other suitable approach. The controller 18 may receive signals from the spinner inlet sensor M1 that are indicative of the one or more properties of fluid flowing into the spinning membrane separator 26 and use the signals to optimize the fluid processing procedure based upon that property or properties. If the property or properties is/are outside of an acceptable range, then the controller 18 may initiate an alarm or error condition to alert an operator to the condition. A suitable device and method for monitoring hematocrit and/or platelet concentration is described in U.S. Patent No. 6,419,822 (which is hereby incorporated herein by reference), but it should be understood that a different approach may also be employed for monitoring one or more properties of a fluid or fluid component flowing into the spinning membrane separator 26.

The illustrated fluid processing device 10 further includes a spinner outlet sensor M2, which accommodates tubing of the fluid flow circuit 12 that flows a separated fluid component out of the spinning membrane separator 26. The spinner outlet sensor M2 monitors the separated fluid component to determine one or more properties thereof, and may do so by optically monitoring the separated fluid component as it flows through the tubing or by any other suitable approach. In one embodiment, separated plasma flows through the tubing, in which case the spinner outlet sensor M2 may be configured to determine the amount of cellular blood components in the plasma and/or whether the plasma is hemolytic and/or lipemic. This may be done using an optical monitor of the type described in U.S. Patent No. 8,556,793 (which is hereby incorporated herein by reference) that measures the optical density of the fluid in the associated tubing, or by any other suitable device and/or method.

As further shown in FIG. 1, the illustrated fluid processing device also includes an air detector M3 (e.g., an ultrasonic bubble detector), which accommodates tubing of the fluid flow circuit 12 that flows fluid to a recipient. It may be advantageous to prevent air from reaching the recipient, whether a human recipient (e.g., the same human that serves as the blood source) or a non-human recipient (e.g., a storage bag or container), so the air detector M3 may transmit signals to the controller 18 that are indicative of the presence or absence of air in the tubing. If the signal is indicative of air being present in the tubing, the controller 18 may initiate an alarm or error condition to alert an operator to the condition and/or to take corrective action to prevent the air from reaching the recipient (e.g., by reversing the flow of fluid through the tubing or diverting flow to a vent location).

The generally vertical portion 24 of the case 20 may include a plurality of volume measurement systems W1-W6 (six are shown, but more or fewer may be provided), each configured to be associated with one or more fluid containers F1-F7 of the fluid flow circuit 12 (FIGS. 2 and 4). Each volume measurement system W1-W6 is configured to work in combination with the controller 18 to measure a current volume of fluid within an associated fluid container F1-F7 and to calculate a change in that volume between two or more points in time. The individual volume measurement systems W1-W6 may be variously configured without departing from the scope of the present disclosure, which may include two or more of the volume measurement systems W1-W6 being differently configured. In one exemplary embodiment, a volume measurement system W1-W6 may be configured as or include a weight scale configured to support and measure the weight of a fluid within an associated fluid container F1-F7 (with the measured weight being converted to a volume by a component of the volume measurement system W1-W6 or by the controller 18). In another exemplary embodiment, a volume measurement system W1-W6 may include one or more sensors configured to detect a volume and/or a change in volume of a fluid within an associated fluid container F1-F7. Volume measurement systems including additional components (e.g., both a weight scale and a sensor) and/or alternative components may also be employed without departing from the scope of the present disclosure. For instance, the volume measurement system can employ flow sensors, optical sensors, or any other suitable sensors without departing from the scope of the disclosure.

Regardless of its particular configuration, each volume measurement system W1-W6 may transmit to the controller 18 a signal that is indicative of the volume of the fluid within the associated container F1-F7 to track the change of volume during the course of a procedure. This allows the controller 18 to process the incremental volume changes to derive fluid processing volumes and flow rates and subsequently generate signals to control processing events based, at least in part, upon the derived processing volumes. For example, the controller 18 may diagnose leaks and obstructions in the fluid flow circuit 12 and alert an operator.

The illustrated case 20 is also provided with a plurality of hooks or supports H1 and H2 that may support various components of the fluid flow circuit 12 or other suitably sized and configured objects.

### D. Controller

According to an aspect of the present disclosure, the fluid processing device 10 includes a controller 18, which is suitably configured and/or programmed to control operation of the fluid processing device 10. In one embodiment, the controller 18 comprises a main processing unit (MPU), which can comprise, e.g., a Pentium^{™} type microprocessor made by Intel Corporation, although other types of conventional microprocessors can be used. In one embodiment, the controller 18 may be mounted inside the generally vertical portion 24 of the case 20, adjacent to or incorporated into an operator interface station (e.g., a touchscreen). In other embodiments, the controller 18 and operator interface station may be associated with the generally horizontal portion 22 or may be incorporated into a separate device that is connected (either physically, by a cable or the like, or wirelessly) to the fluid processing device 10.

The controller 18 is configured and/or programmed to execute at least one fluid processing procedure but, more advantageously, is configured and/or programmed to execute a variety of different fluid processing procedures. For example, the controller 18 may be configured and/or programmed to carry out one or more of the following: a double unit red blood cell collection procedure, a plasma collection procedure, a plasma/red blood cell collection procedure, a red blood cell/platelet/plasma collection procedure, a platelet collection procedure, and a platelet/plasma collection procedure.

More particularly, in carrying out these fluid processing procedures, the controller 18 is configured and/or programmed to control one or more of the following tasks: drawing fluid into a fluid flow circuit 12 mounted to the fluid processing device 10, conveying fluid through the fluid flow circuit 12 to a location for separation (i.e., into a spinning membrane separator 26 or centrifugal separation chamber 36 of the fluid flow circuit 12), separating the fluid into two or more components as desired, and conveying the separated components into storage containers, to a second location for further separation (e.g., into whichever of the spinning membrane separator 26 and centrifugal separation chamber 36 that was not used in the initial separation stage), or to a recipient (which may be a source from which the fluid was originally drawn).

This may include instructing the spinning membrane separator drive unit 14 and/or the centrifugal separator 16 to operate at a particular rotational speed and instructing a pump P1-P6 to apply a particular (positive or negative) pressure to a flexible membrane or diaphragm of an associated pump chamber T1-T6 of the cassette 48 for a particular duration to convey fluid through a portion of the fluid flow circuit 12 at a particular flow rate. Hence, while it may be described herein that a particular component of the fluid processing device 10 (e.g., the spinning membrane separator drive unit 14 or the centrifugal separator 16) performs a particular function, it should be understood that that component is being controlled by the controller 18 to perform that function.

Before, during, and after a procedure, the controller 18 may receive signals from various components of the fluid processing device 10 (e.g., the pressure sensors A1-A4) to monitor various aspects of the operation of the fluid processing device 10 and characteristics of the fluid and separated fluid components as they flow through the fluid flow circuit 12. If the operation of any of the components and/or one or more characteristics of the fluid or separated fluid components is outside of an acceptable range, then the controller 18 may initiate an alarm or error condition to alert the operator and/or take action to attempt to correct the condition. The appropriate corrective action will depend upon the particular error condition and may include action that is carried out with or without the involvement of an operator.

For example, the controller 18 may include an interface control module, which receives signals from the light detector 52 of the interface monitoring system. The signals that the controller 18 receives from the light detector 52 are indicative of the location of an interface between the separated fluid components within the centrifugal separation chamber 36. If the controller 18 determines that the interface is in the wrong location, then it can issue commands to the appropriate components of the fluid processing device 10 to modify their operation so as to move the interface to the proper location. For example, the controller 18 may instruct one of the pumps P1-P6 to cause fluid to flow into the centrifugal separation chamber 36 at a different rate and/or for a separated fluid component to be removed from the centrifugal separation chamber 36 at a different rate and/or for the centrifugal separation chamber 36 to be spun at a different speed by the centrifugal separator 16.

If provided, an operator interface station associated with the controller 18 allows the operator to view on a screen or display (in alpha-numeric format and/or as graphical images) information regarding the operation of the system. The operator interface station also allows the operator to select applications to be executed by the controller 18, as well as to change certain functions and performance criteria of the system. If configured as a touchscreen, the screen of the operator interface station can receive input from an operator via touch-activation. Otherwise, if the screen is not a touchscreen, then the operator interface station may receive input from an operator via a separate input device, such as a computer mouse or keyboard. It is also within the scope of the present disclosure for the operator interface station to receive input from both a touchscreen and a separate input device, such as a keypad.

### II. The Disposable Fluid Flow Circuit

As for the fluid flow circuit or flow set 12 (FIG. 2), it is intended to be a sterile, single use, disposable item. Before beginning a given fluid processing procedure, the operator mounts various components of the fluid flow circuit 12 to the case 20 in association with the fluid processing device 10. The controller 18 implements the procedure based upon preset protocols, taking into account other input from the operator. Upon completing the procedure, the operator removes the fluid flow circuit 12 from association with the fluid processing device 10. The portions of the fluid flow circuit 12 holding the collected fluid component or components (e.g., collection containers or bags) are removed from the case 20 and retained for storage, transfusion, or further processing. The remainder of the fluid flow circuit 12 is removed from the case 20 and discarded.

In the illustrated embodiment, the fluid flow circuit 12 includes a cassette 48 (FIG. 3), to which the other components of the fluid flow circuit 12 are connected by flexible tubing. The other components may include a plurality of fluid containers F1-F7. In the context of the present disclosure, these containers include an anticoagulant container F1, a saline container F2, an in-process container F3, a return container F4, a plasma collection container F5, a platelet collection container F6, and an (optional) additive container F7. The illustrated flow circuit 12 further includes one or more fluid source access devices (e.g., a connector for accessing blood within a fluid container or a phlebotomy needle), a spinning membrane separator 26 and a centrifugal separation chamber 36.

The flow control cassette 48 provides a centralized, programmable, integrated platform for all the pumping and many of the valving functions required for a given fluid processing procedure. In one embodiment, the cassette 48 is similarly configured to the cassette of U.S. Patent Application Publication No. 2006/0161092, but is adapted to include the various stations and flow paths required to carry out the fluid processing procedures implemented by the fluid processing system.

In use, the cassette 48 is mounted to the cassette station 54 of the fluid processing device 10, with a flexible membrane or diaphragm of the cassette 48 placed into contact with the cassette station 54. The flexible diaphragm overlays an array of interior cavities formed by the body of the cassette 48. The different interior cavities define sensor stations S1-S4, valve stations C1-C9, pump stations T1-T6, and a plurality of flow paths. The side of the cassette 48 opposite the flexible diaphragm may be sealed by another flexible diaphragm or by a rigid cover, thereby sealing fluid flow through the cassette 48 from the outside environment.

Each sensor station S1-S4 is aligned with an associated pressure sensor A1-A4 of the cassette station 54, with each pressure sensor A1-A4 being capable of monitoring the pressure within the associated sensor station S1-S4. Each valve station C1-C9 is aligned with an associated valve V1-V9 and may define one or more ports that allow for fluid communication between the valve station C1-C9 and another interior cavity of the cassette 48 (e.g., a flow path). As described above, each valve V1-V9 is movable under command of the controller 18 to move between a plurality of positions (e.g., between a retracted or lowered position and an actuated or raised position) to selectively contact the valve stations C1-C9 of the cassette 48. In the actuated position, a valve V1-V9 engages the associated valve station C1-C9 to close one or more of its ports to prevent fluid flow therethrough. In the retracted position, a valve V1-V9 is disengaged from the associated valve station C1-C9 (or less forcefully contacts the associated valve station C1-C9 than when in the actuated position) to open one or more ports associated with the valve station C1-C9, thereby allowing fluid flow therethrough.

As described above, the cassette 48 defines a plurality of pump chambers T1-T6, with each pump chamber interacting with a different one of the pneumatic pumps P1-P6 of the cassette station 54 of the fluid processing device 10. The different pumps P1-P6 may interact with the pump stations T1-T6 of the cassette 48 to perform different tasks during a procedure, but in the context of the present disclosure, a different one of the pumps P1-P6 may be configured to serve as an anticoagulant pump P1, a source pump P2, a centrifuge pump P3, an outlet pump P4, a recirculation pump P5, and a plasma pump P6.

Various lengths of tubing extend from the side surface of the cassette 48 to connect to the other components of the fluid flow circuit 12, such as the various fluid containers F1-F7, the spinning membrane separator 26, and the centrifugal separation chamber 36. The tubing connected to the centrifugal separator chamber 36 (which includes one inlet tube and two outlet tubes) may be aggregated into an umbilicus.

Various additional components may be incorporated into the tubing leading out of the cassette 48 or into one of the cavities of the cassette 48. For example, as shown in FIG. 2, a manual clamp 56 may be associated with a line or lines leading to the fluid source, a return line filter 58 (e.g., a microaggregate filter) may be associated with a line leading to a fluid recipient, and/or an air trap 62 may be positioned on a line upstream of the centrifugal separation chamber 36.

### III. Exemplary Fluid Processing Procedure

An exemplary fluid processing procedure according to the present disclosure will now be described. In the exemplary procedure, blood is separated via centrifugation into packed red blood cells and platelet-rich plasma, with a portion of the platelet-rich plasma being recirculated back through a centrifugal separation chamber and another portion being separated into platelet concentrate and platelet-poor plasma until a target volume of platelets has been collected. It should be understood that the below-described procedure is merely exemplary and that the principles described herein may be practiced in combination with other fluid processing procedures (e.g., procedures in which platelet-poor plasma is recirculated through a centrifugal separation chamber during blood separation) without departing from the scope of the present disclosure.

Prior to processing, an operator selects the desired protocol (e.g., using an operator interface station, if provided), which informs the controller 18 of the manner in which it is to control the other components of the fluid processing device 10 during the procedure. This may include first selecting one of a plurality of possible procedures that the system is capable of executing and then, after selecting the nature of the procedure, selecting one or more parameters to be in effect during the procedure. For example, this may include selecting a platelet collection procedure from among a variety of blood separation procedures and then selecting a total volume of blood to be processed or a target volume of platelets to be collected during the procedure. If the fluid source is a living source/subject (e.g., a donor or patient), the operator may proceed to enter various parameters, such as the sex/height/weight of the source. In one embodiment, the operator may also enter one or more characteristics of the fluid to be processed, such as a platelet pre-count.

If there are any fluid containers (e.g., a platelet additive solution container) that are not integrally formed with the fluid flow circuit 12, they may be connected to the fluid flow circuit 12 (e.g., by piercing a septum of a tube of the fluid flow circuit 12 or via a luer connector), with the fluid flow circuit 12 then being mounted to the fluid processing device 10 (including the fluid containers F1-F7 being associated with the volume measurement systems W1-W6, as appropriate). In one exemplary embodiment, each volume measurement system W1-W6 includes a weight scale associated with a hook from which a fluid container may be hung. In another exemplary embodiment, at least one of the volume measurement systems W1-W6 includes a weight scale associated with a horizontal platform or surface, with a container being placed onto the platform or surface for support while the weight scale sends signals indicative of the weight of the container (and its contents) to be sent to the controller 18 throughout the course of a procedure. In other embodiments, a fluid container may be associated with a volume measurement system omitting a weight scale, but including other means for measuring the volume of fluid within the container (e.g., one or more sensors, such as a flow sensor or optical sensor).

An integrity check of the fluid flow circuit 12 may be executed by the controller 18 to ensure that the various components are properly connected and functioning. Following a successful integrity check, the fluid source is connected to the fluid flow circuit 12 (e.g., by connecting to a container of previously collected fluid or by phlebotomizing a subject), and the fluid flow circuit 12 may be primed (e.g., using saline pumped from a saline container F2 by operation of one or more of the pumps P1-P6 of the fluid processing device 10).

After the fluid flow circuit 12 has been primed, fluid processing may begin. In a first phase of an exemplary platelet collection procedure (FIG. 4), blood is drawn into the fluid flow circuit 12 from a blood source. If the blood source is a subject, then blood may be drawn into the fluid flow circuit 12 through a single needle that is connected to the cassette by line L1. Line L1 may include a manual clamp 56 that may initially be in a closed position to prevent fluid flow through line L1. When processing is to begin, an operator may move the manual clamp 56 from its closed position to an open position to allow fluid flow through line L1.

The blood is drawn into line L1 by the source pump P2 of the fluid processing device 10. Anticoagulant from the anticoagulant container F1 may be drawn through line L2 under action of the anticoagulant pump P1 and added to the blood at a junction of lines L1 and L2.

In the illustrated embodiment, valve V10 is open to allow anticoagulated blood to flow through line L3 and a cassette sensor station associated with pressure sensor A1, while valve V11 is closed to prevent fluid flow through line L4. If the blood source is a living body (e.g., a subject), the pressure sensor A1 may communicate with the controller 18 to monitor the pressure within the vein of the blood source.

The cassette includes two valve stations downstream of the source pump P2, with valve V2 being closed to prevent flow through line L5 and valve V1 being open to allow flow through line L6. A portion of the blood is directed through line L7 and a cassette sensor station associated with pressure sensor A3 to the in-process container F3 and the remainder is directed through line L8 toward the centrifuge pump P3, which controls the amount of blood that is directed to the centrifugal separation chamber 36 instead of the in-process container F3. In particular, the flow rate of the source pump P2 is greater than the flow rate of the centrifuge pump P3, with the difference therebetween being equal to the flow rate of blood into the in-process container F3. The flow rates may be selected such that the in-process container F3 is partially or entirely filled with blood at the end of the draw phase.

The blood pumped through line L8 by the centrifuge pump P3 passes through line L9, an air trap 62, and a cassette sensor station associated with pressure sensor A2 (which works in combination with the controller 18 of the fluid processing device 10 to monitor the pressure in the centrifugal separation chamber 36) before reaching the centrifugal separation chamber 36 of the fluid flow circuit 12. The centrifugal separator 16 of the fluid processing device 10 manipulates the centrifugal separation chamber 36 to separate the blood in the centrifugal separation chamber 36 into platelet-rich plasma and packed red blood cells. In one embodiment, the centrifugal separation chamber 36 is rotated nominally at 4,500 rpm, but the particular rotational speed may vary depending on the flow rates of fluids into and out of the centrifugal separation chamber 36.

The packed red blood cells exit the centrifugal separation chamber 36 via line L10 and flow through line L11 into the return container F4. Platelet-rich plasma is drawn out of the centrifugal separation chamber 36 via line L12 by the combined operation of the recirculation and outlet pumps P5 and P4 of the fluid processing device 10. The platelet-rich plasma travels through line L12 until it reaches a junction, which splits into lines L13 and L14. The recirculation pump P5 is associated with line L13 and redirects a portion of the platelet-rich plasma to a junction, where it mixes with blood in line L8 that is being conveyed into the centrifugal separation chamber 36 by the centrifuge pump P3. Recirculating a portion of the platelet-rich plasma into the centrifugal separation chamber 36 with inflowing blood decreases the hematocrit of the blood entering the centrifugal separation chamber 36, which may improve separation efficiency. By such an arrangement, the flow rate of the fluid entering the centrifugal separation chamber 36 is equal to the sum of the flow rates of the centrifuge pump P3 and the recirculation pump P5. As the platelet-rich plasma drawn out of the centrifugal separation chamber 36 into line L13 by the recirculation pump P5 is immediately added back into the centrifugal separation chamber 36, the bulk or net platelet-rich plasma flow rate out of the centrifugal separation chamber 36 is equal to the flow rate of the outlet pump P4.

Line L14 ends at a junction, where it joins with lines L15 and L16. Valve V6 is closed to prevent fluid flow through line L16, thereby directing the separated platelet-rich plasma to the spinning membrane separator 26 via line L15. Before reaching the spinning membrane separator 26, the portion of the platelet-rich plasma conveyed through line L15 passes the spinner inlet sensor M1 and a cassette sensor station associated with pressure sensor A4. The spinner inlet sensor M1 may detect the concentration of platelets in the platelet-rich plasma entering the spinning membrane separator 26, while the pressure sensor A4 may monitor the pressure of the spinning membrane separator 26.

While valve V6 is shown in FIG. 4 as being closed, it may be selectively opened to divert all or a portion of the platelet-rich plasma from line L14 into and through line L16 and to the return container F4, if necessary. An example would be at the start of a procedure when separation is initializing and platelets are not yet exiting the centrifugal separation chamber 36, in which case the fluid conveyed through line L14 by the outlet pump P4 could be diverted to the return container F4.

The spinning membrane separator drive unit 14 of the fluid processing device 10 manipulates the spinning membrane separator 26 to separate the platelet-rich plasma into platelet-poor plasma ("plasma") and platelet concentrate ("platelets"). Plasma is pumped out of the spinning membrane separator 26 via line L17 by the plasma pump P6 of the fluid processing device 10. Valves V5, V6, V8, and V9 are closed to direct the separated plasma along line L18, through valve V4, and into the return container F4 (with the separated red blood cells). On the way to the return container F4, the plasma passes through spinner outlet sensor M2, which may cooperate with the controller 18 to determine one or more characteristics of the plasma, such as the amount of cellular blood components in the plasma and/or whether the plasma is hemolytic and/or lipemic.

The platelet concentrate is conveyed out of the spinning membrane separator 26 via line L19. There is no pump associated with line L19, so instead the flow rate at which the platelets exit the spinning membrane separator 26 is equal to the difference between the flow rates of the outlet pump P4 and plasma pump P6. Valve V8 is closed to prevent fluid flow through the line L20, thereby directing the flow of platelets along line L19, through valve V7, and into the platelet collection container F6. Valve V8 may be selectively opened to allow fluid flow through line L20 and to a junction, where it joins the plasma flowing through line L18 to the return container F4, if necessary.

Depending on the volume of platelets to be collected, the draw stage of FIG. 4 may be repeated, with draw stages being alternated with return stages in which blood from the in-process container F3 is separated in the centrifugal separation chamber 36 while previously collected blood components in the return container F4 are returned to the blood source. During such return stages, the separated red blood cells and platelet-rich plasma may be variously routed through the fluid flow circuit 12, typically with an additional volume of platelets being collected in the platelet collection container F6 after being separated from platelet-poor plasma in the spinning membrane separator 26 (as during the draw stage of FIG. 4). A platelet additive solution from the additive container F7 may be added to the collected platelets in the platelet collection container F6 before ending the procedure.

### IV. Fluid Flow Rate Optimizer

In an embodiment, controller 18 includes a fluid flow rate optimizer. For instance, controller 18 is configured to determine an optimum flow rate for flowing a fluid through the disposable circuit 12, such as from a fluid container. In an example, the controller 18 is configured to determine an optimum flow rate for reinfusing a return fluid to a subject. By optimizing the flow rate of fluid from a fluid container, the time a subject is connected to the blood processing device can be shortened. For illustrative purposes, methods for determining the optimum flow rate for flowing a fluid through a circuit will be described in the context of reinfusing a return fluid to a subject as executed by controller 18 using device 10 and fluid flow circuit 12, but it will be understood that the methods can be carried out on differently configured systems and devices without departing from the scope of the disclosure.

The fluid can be blood, a separated blood component, or any suitable return fluid (e.g., saline). In a particular example, the return fluid can be RBCs separated from whole blood during a blood processing procedure. The return fluid can be the collected blood components in the return container F4.

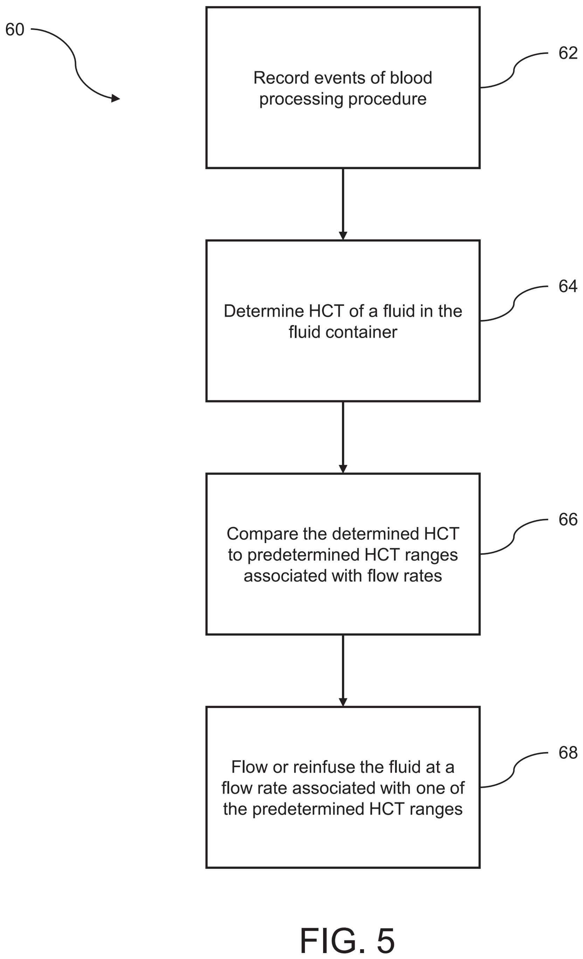

An example of a method 60 for determining an optimum flow rate for flowing a fluid from a fluid container is shown in FIG. 5. For instance, controller 18 can be configured to execute method 60 to determine the optimum flow rate by recording the events of a blood processing procedure (block 62), determining the hematocrit of the fluid in the fluid container (block 64), and comparing the determined fluid hematocrit to predetermined reference hematocrit ranges associated with flow rates (block 66). The fluid is then flowed from the fluid container at the flow rate associated with the predetermined reference hematocrit range in which the determined hematocrit falls (block 68).

As described herein, controller 18 may receive signals from various components (e.g., sensors) of the fluid processing device 10 to monitor various aspects of the fluid processing device 10 and characteristics of the fluid and separated fluid components as they flow through the fluid flow circuit 12. Additionally, in some embodiments, controller 18 can be configured to record blood processing system events during a blood processing procedure, including, but not limited to, air purges and centrifuge inlet occlusions. In an example, recording events can include recording the event and when the event occurred during the blood processing procedure. For instance, the controller 18 can record the event and the stage of the blood processing procedure during which the event occurred (e.g., collection, reinfusion, etc.). In a particular example, the time elapsed during a particular stage can be recorded with the event. Using the recorded information, controller 18 can determine the hematocrit or a hematocrit range of the return fluid.

In an example, method 60 can be used to determine the optimum flow rate to reinfuse a return fluid from a return container. To determine the hematocrit of the return fluid, the controller 18 can be configured to know that particular blood components included in the return fluid have a hematocrit range (i.e., predetermined hematocrit values of particular blood components can be stored in the memory of controller 18). For example, if the return fluid includes RBCs, the controller 18 can be configured to know that packed red blood cells can have a hematocrit range from about 78%-82%. The ranges can vary without departing from the scope of the disclosure depending on the efficiency of the procedure.

The controller 18 can then determine a return fluid hematocrit range based on the predetermined hematocrit ranges of the blood components present in the return fluid, while taking into account any recorded event during the blood processing procedure. In particular, controller 18 can determine the return fluid hematocrit range based on a recorded event that could have introduced or removed fluid from the return container F4, such as, but not limited to, an air purge.

An air purge may be required to remove air (e.g., an air bubble) detected within the return line to prevent air from returning to a subject. For instance, an air purge can occur when the system detects an air bubble in the return line or an operator can direct the controller 18 to execute an air purge. In some instances, multiple air purges may be required to remove the air from the return line.

In an example, during an air purge, whole blood can be used to displace air in the return line past the junction where saline from saline container F2 meets the return line, at which point the saline can be used to displace the air into the return container F4 to minimize the volume of whole blood pulled from the subject. In another example, air can be purged using only whole blood. Controller 18 can be configured to know that a recorded air purge can add a volume of whole blood and/or saline to the return container F4. In an example, an air purge may add about 5 mLs of whole blood and 10 mLs of saline to the return container F4, yet the volume of whole blood and saline used in an air purge can vary without departing from the scope of the disclosure. By knowing and recording the volume of additional fluid added to the return container F4, controller 18 can determine the return fluid hematocrit range.

Other recorded events that could be used to determine the hematocrit range include, but are not limited to, centrifuge inlet line occlusions, manual saline reinfusion, draw/return stage transitions, centrifuge spin downs, product target changes, subject parameter changes, procedure settings changes (e.g., AC ratio changes), detection of an empty saline container, addition of a new saline bag, and procedure progress events (i.e., blood prime completion or other state transitions).

After determining a return fluid hematocrit range, controller 18 can be configured to compare the return fluid hematocrit range with predetermined reference hematocrit ranges, which are associated with different flow rates. For example, predetermined reference hematocrit ranges can be stored in the memory of controller 18. Each reference hematocrit range can be associated with a different flow rate. For instance, a first reference hematocrit range is associated with a first flow rate, a second reference hematocrit range is associated with a second flow rate, and a third reference hematocrit range is associated with a third flow rate, etc. In an example, a reference hematocrit range of 0-10% can be associated with a flow rate of up to 300 mL/min. In another example, a reference hematocrit range of 45-55% can be associated with a flow rate of up to 150 mL/min. In another example, a reference hematocrit range of 75-85% can be associated with a flow rate of about 60 mL/min. Without departing from the scope of the disclosure, the number of predetermined reference hematocrit ranges and their breadth can vary and can span all possible hematocrit values that the return fluid can have. Additionally, the flow rates associated with the reference hematocrit ranges can vary and can be any suitable flow rates without departing from the scope of the disclosure.

In a particular example, the controller 18 is configured to compare the lowest value of the determined return fluid hematocrit range to the reference hematocrit ranges. After determining within which predetermined reference hematocrit range the lowest value of the determined return fluid hematocrit range falls, controller 18 directs system 10 to pump the return fluid to the subject at the flow rate associated with that predetermined reference hematocrit range.

In some examples, system 10 can include sensors configured to determine the return fluid hematocrit. For instance, sensors (e.g., optical sensors) can be arranged on device 10 to monitor the fluid entering return container F4. Information from the sensors can be used in addition to or in lieu of recorded events (block 62) to determine the return fluid hematocrit. The measured return fluid hematocrit can then be compared to the predetermined reference hematocrit ranges associated with return flow rates. After determining within which predetermined reference hematocrit range the determined return fluid hematocrit falls, controller 18 can direct device 10 to pump the return fluid to the subject at the flow rate associated with that predetermined reference hematocrit range.

In another example, sensors, such as, but not limited to optical sensors, can be arranged on device 10 to monitor the fluid exiting return container F4. The sensors can be configured to determine the hematocrit of the fluid exiting return container F4. The determined hematocrit of the return fluid can be compared to the pre-determined reference hematocrit ranges (block 66) and returned at the flow rate associated with the pre-determined reference hematocrit range in which the return fluid hematocrit falls (block 68).

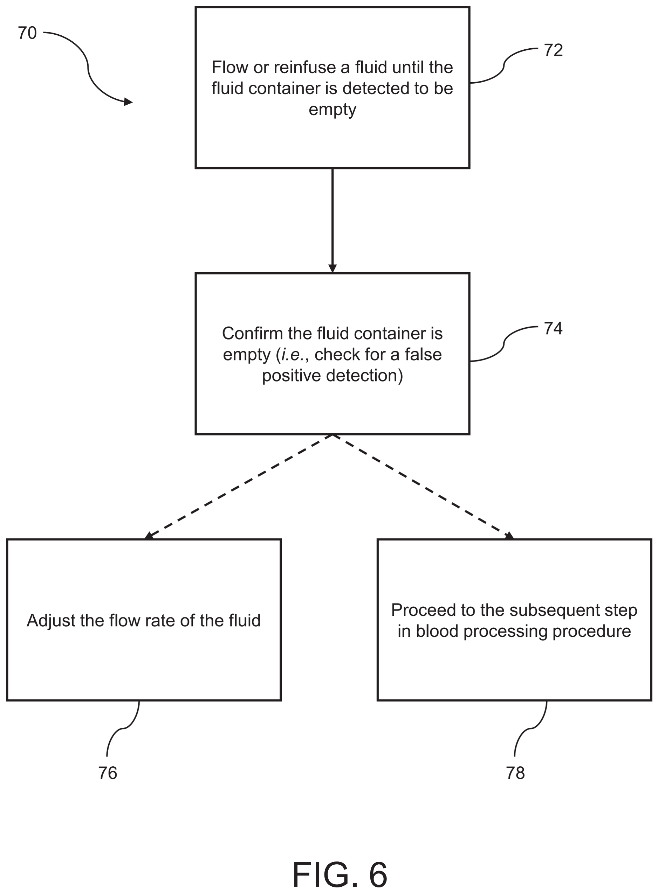

Turning to FIG. 6, the figure illustrates another method 70 for determining an optimum flow rate for flowing a fluid from a fluid container. The controller 18 can execute method 70 by being configured to flow a fluid from a fluid container until the fluid container is detected to be empty (block 72), confirm if the fluid container is empty (i.e., check for a false positive empty detection) (block 74), and optionally, adjust the flow rate of the fluid (block 76). In an example, if the controller 18 confirms that the fluid container is empty, the method 70 can proceed to the subsequent step in the blood processing procedure (block 78). In an example, method 70 is employed to determine the optimum flow rate at which to reinfuse a return fluid from a return container to a subject.

In an example, the controller 18 can be configured to reinfuse return fluid at the highest suitable flow rate. For example, the highest suitable flow rate can be a flow rate up to a predetermined limit flow rate. In some embodiments, an operator can input the predetermined limit flow rate to the system (i.e., the controller 18 receives the predetermined limit flow rate from the operator). Alternatively, controller 18 can be configured to determine the highest suitable flow rate at which to reinfuse return fluid. As described herein, controller 18 may receive signals from various components of the fluid processing device 10 to monitor various aspects of the fluid processing device 10 and characteristics of the fluid and separated fluid components as they flow through the fluid flow circuit 12. Controller 18 can determine the highest suitable flow rate at which to reinfuse return fluid based on various information including, but not limited to, the recorded information received by the controller 18, subject information inputted by the device operator (e.g., sex, height, and weight), a predetermined limit flow rate inputted by an operator, flow rate restrictions of the pump (i.e., the maximum flow rate of the pump), the rate at which citrate can safely be returned to a subject (i.e., CIR, which can be determined by the subject information), intelligent flow control (IFC), which can limit the flow rate to prevent vein issues, and/or limitations on the flow rates as set by the operator per standard operating procedures.

Controller 18 can be configured to reinfuse the return fluid at the highest suitable rate until the device 10 detects that the return fluid container F4 is empty. Controller 18 may receive signals from various components (e.g., sensors) of the fluid processing device 10 to determine if the return container F4 is empty. In some embodiments, return container F4 can be associated with the volume measurement system W1-W6, which can send a signal to the controller 18 when return container F4 is detected to be empty. In particular, the weight and/or volume of return container F4 can be determined using the weight scales and sensors as described herein. Alternatively, controller 18 can determine that return container F4 is empty by detecting that the weight and/or volume of the return container F4 has not changed for a period of time after reinfusion has begun, by tracking the flow rate and amount of elapsed time during reinfusion, or by using any other suitable sensors or trackers.

Additionally, controller 18 is configured to confirm that a fluid container of the disposable circuit 12 is empty. For instance, after the controller 18 receives a signal that a container is empty, the controller is configured to confirm that the container has been correctly detected as empty. Unless otherwise described, it will be understood that empty herein is used to describe a container that is completely empty or sufficiently empty such that only a minimal amount of fluid remains in the container.

In an example, controller 18 is configured to confirm that return container F4 is empty. In particular, after initially detecting that the return container F4 is empty, controller 18 is configured to determine if the empty detection is a false positive as described in greater detail herein. If the return container 18 is confirmed to be empty, the procedure can proceed to a subsequent step in the procedure (block 78), for instance, disconnecting the subject from the fluid circuit 12. If it is determined that the return container still contains return fluid (i.e., a false positive empty detection), the controller 18 and/or operator can take a corrective action, and the controller 18 can subsequently resume reinfusing the subject with the return fluid. In an example, the controller 18 can adjust the flow rate to a lower rate (block 76). Decreasing the flow rate minimizes the risk of another false positive reading during reinfusion, thus shortening the time a subject is connected to the system. In some examples, the flow rate can be adjusted in predetermined increments. Alternatively, controller 18 can be configured to determine a suitable adjustment to the flow rate.

To determine if the empty detection is a false positive, controller 18 can utilize signals from the various sensors and scales associated with device 10 and/or pumps associated with device 10 to flow fluids through portions of the circuit 12.

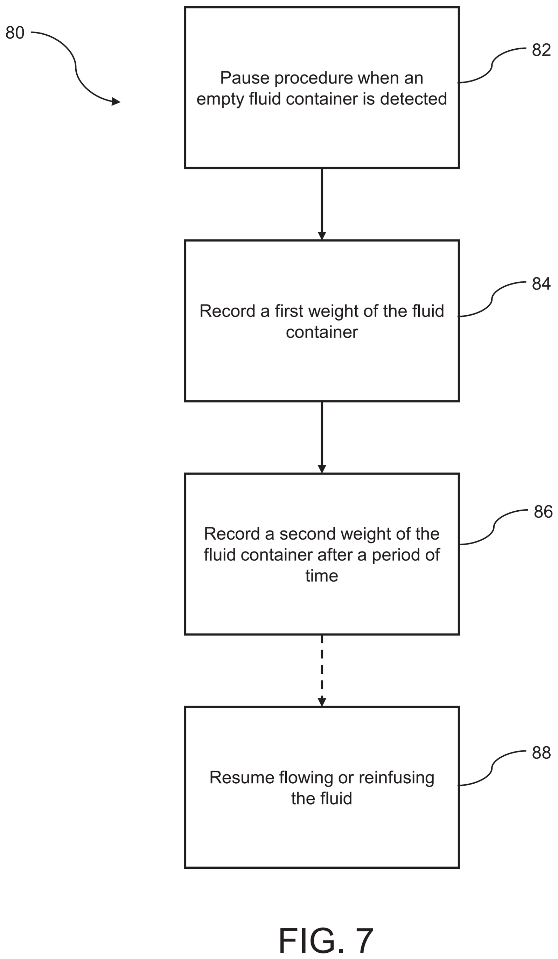

In an example, FIG. 7 shows a method 80 for determining if the empty detection is a false positive. As shown in FIG. 7, the controller 18 can determine a false positive detection by being configured to pause the procedure after a fluid container is detected to be empty (block 82) and to monitor a change of volume or weight in the fluid container. In particular, controller 18 is configured to record a first weight of the fluid container after the controller 18 determines that the fluid container is empty (block 84) and then record a second weight of the fluid container after a period of time has elapsed (block 86). In an example, controller 18 can measure the weight of fluid container using signals from weight management system W1-W6.

In an example, the fluid container can be the return container F4 and the fluid can be a return fluid. In some instances, the return container F4 may be initially determined to be empty because of a collapsed portion of the fluid pathway carrying the return fluid. If a portion of the pathway collapses, for instance due to the flow rate of return fluid being too high, creating a negative pressure in the tubing, given the head height created by the return container F4 on the device 10, a volume from the return container F4 will flow into the collapsed pathway once the pump reinfusing the return fluid stops pumping.

When the controller 18 detects the return container F4 is empty (for example, due to a collapsed portion of the fluid pathway), the pump stops and a first weight of the return fluid container F4 is recorded, and after a period of time, a second weight of the return container F4 is recorded. If the weight of the return container F4 decreases or decreases by an amount greater than a predetermined threshold value, the return container F4 was not emptied (i.e., fluid in the return container F4 replaced the negative pressure in the pathway created by the too fast pump). Conversely, if the weight of the return container F4 does not change (or decreases, but not greater than a predetermined threshold value), the return container F4 was sufficiently emptied. If a false positive is determined, the controller 18 can be configured to resume reinfusion (block 88) by restarting the pump at the same or at an adjusted lowered flow rate. In an example, after restarting reinfusion after determining a false positive detection, the controller can direct the pump to continue pumping for a predetermined amount of time before repeating the test.

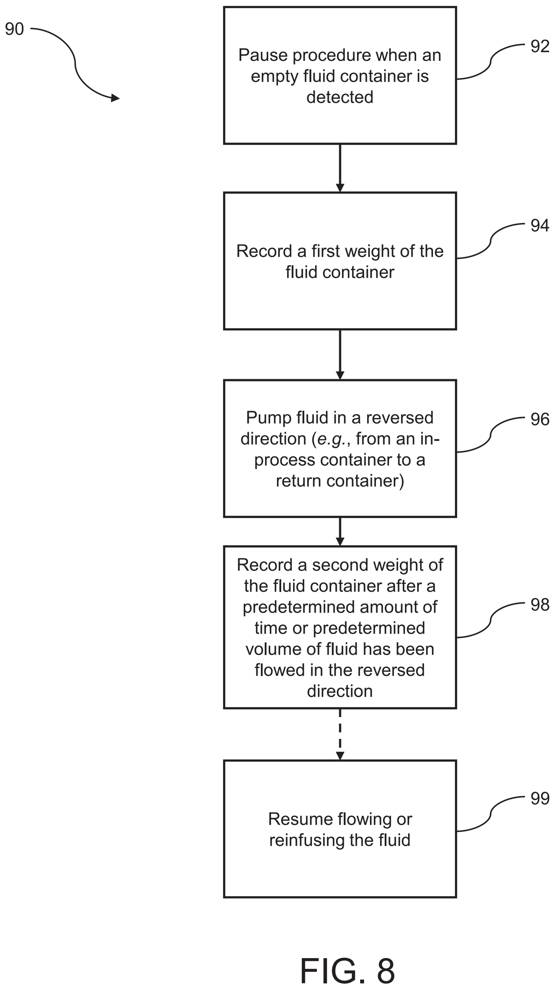

FIG. 8 shows another example of a method 90 for determining if a false positive empty detection occurred. As shown in FIG. 8, the controller 18 can determine a false positive by being configured to pause the blood processing procedure when a fluid container is detected to be empty (block 92), record a first weight of the fluid container (block 94), pump a volume of fluid in a reversed direction (block 96), and record a second weight of the fluid container after pumping the volume of fluid in the reversed direction (block 98).

In an example, controller 18 can be configured to pump return fluid, such as, but not limited to, RBCs, from the return container F4 to an in-process container F3. Similarly to the method of FIG. 7, when the controller 18 detects that the return container F4 is empty, the procedure is paused (block 92) and a first weight of the return container F4 is recorded (block 94). At this point the controller 18 directs a pump to pump in a reversed direction, directing fluid from the in-process container F3 towards the return container F4 for a predetermined time or volume (block 96), replacing the volume vacated by an overly fast reinfusion rate into return container F4. A second weight of the return container F4 is recorded after the pump has pumped in the reversed direction for the predetermined amount of time or volume (block 98). If the weight of the return container F4 does not change after pumping in the reversed direction for the predetermined amount of time or volume (or changes less than a predetermined threshold value), the return container F4 is confirmed to be empty. If the second recorded weight is greater than the first recorded weight, a false positive is confirmed. In an example, if the second recorded weight is greater than the first recorded weight, a false positive is confirmed if the difference between the second weight and first weight is greater than a predetermined threshold value.

In a particular example, after the controller 18 detects that return container F4 is empty, a first weight of return container F4 is recorded and pump P2 pumps in the reverse direction, displacing any fluid (if present) from L5 and L11 towards return container F4. After pumping a predetermined volume or for a predetermined amount of time, a second weight of return container F4 is recorded. If the weight of return container F4 increases, and the difference between the second recorded weight and the first recorded weight is greater than a predetermined threshold value, a false positive is confirmed. The controller 18 can then resume reinfusion by directing the pump to restart reinfusion at the same rate or at an adjusted lowered flow rate (block 99).

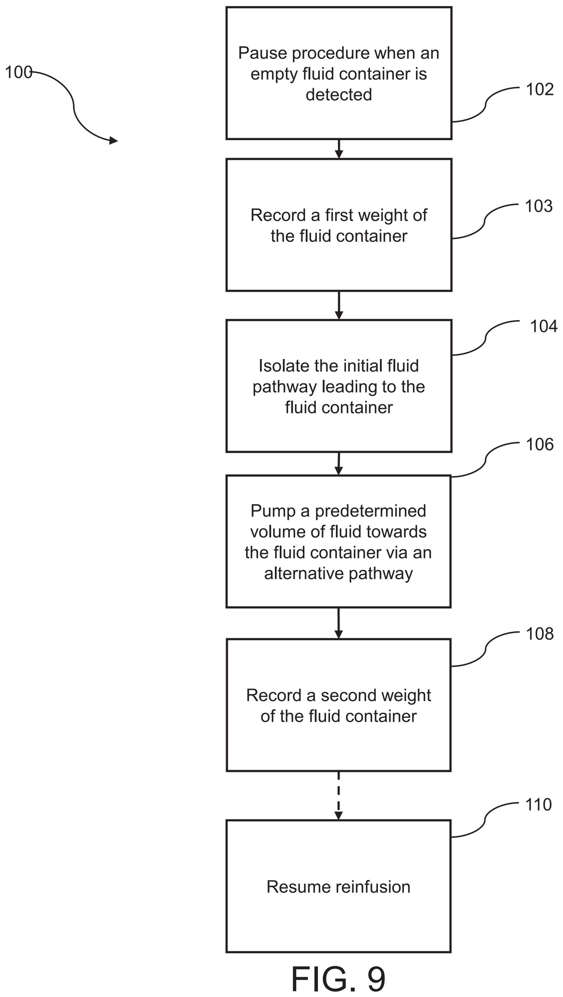

In yet another example, FIG. 9 shows a method 100 for determining a false positive empty detection by using an alternative pathway. In this instance, the controller 18 can determine a false positive by being configured to attempt to refill the fluid container by utilizing a different fluid pathway. The controller is configured to pause the procedure when the fluid container is detected to be empty (block 102), record a first weight of the fluid container (block 103), isolate the initial fluid pathway leading to the fluid container (e.g., the pathway used to fill the fluid container) (block 104), pump a predetermined volume of fluid towards the fluid container via an alternative pathway (block 106), and record a second weight of the fluid container after pumping the predetermined volume of fluid (block 108).

In an example, a false positive detection that the return container F4 is empty can be confirmed by utilizing method 100. For instance, after return container F4 has been detected to be empty and the procedure is paused (block 102), a first weight of the return container F4 is recorded (block 103) and the pathway leading to the return container F4 (i.e., the pathway through which fluid had been previously pumped) is isolated (block 104). In an example, return fluid exits the return container F4 via line L11, line L5, and valve V2. After return container F4 has been detected to be empty, the pathway can be isolated by closing valve V2. Accordingly, by isolating the pathway, the negative pressure of a possible collapsed pathway is isolated.

The controller 18 is configured to then direct a pump to pump a predetermined volume in the direction of the return container F4 through an alternative pathway (block 106) and to record a second weight of the return container F4 (block 108). In an example, fluid from L18 can be pumped through V4 and into L11, yet other alternative pathways can be used without departing from the scope of the disclosure. Alternatively, the weight of return container F4 can be continuously monitored as the predetermined volume is pumped through the alternative pathway.

The predetermined volume pumped through the alternative pathway can be selected such that it will only re-prime a portion of the alternative pathway leading to the return container F4. Accordingly, if the initial pathway leading to the return container F4 was properly emptied, isolating the initial pathway and pumping the volume through the alternative pathway results in a small or no change in the weight of the return container F4. If the difference in weight of the return container F4 exceeds a predetermined threshold value, a false positive is confirmed. In an example, if the weight of the return container exceeds a percentage of a weight of the volume of fluid pumped towards the return fluid container F4 through the alternative pathway, a false positive is confirmed. In other words, if the weight in return container F4 changes beyond a predetermined threshold percentage of the amount of fluid pumped, fluid within the original pathway was not emptied. For instance, fluid within line L11 was displaced back into the return container F4 by the fluid being pumped via the alternative pathway. If a false positive is confirmed, the controller 18 can resume reinfusion (block 110) by directing the pump to restart at the same rate or at an adjusted lowered flow rate.

The controller 18 can be configured to execute one of or any combination of the methods for determining an optimum flow rate 60 and 70 and to determine a false positive 80, 90, and 100 as described herein. In an example, controller 18 can be configured to determine which method 60 or 70 would provide a quickest suitable blood processing procedure. For instance, controller 18 can be configured to prioritize method 70 and if method 70 is determined to not be suitable (i.e., not viable due to the configuration of the system or not the fastest/most efficient procedure), method 60 can be executed. Additionally, controller 18 can be configured to determine a suitable method for determining a false positive. For instance, controller 18 can be configured to evaluate the procedure to determine which method of determining a false positive is quickest. In a particular example, controller 18 is configured to determine a false positive by attempting to refill the return container by utilizing a different fluid pathway (method 100), yet if multiple pathways are not possible, controller 18 can attempt to refill the return container by reversing fluid flow (method 90) or pause the procedure and monitor a change of volume or weight in the return container (method 80), whichever is quicker.

Although the methods for determining an optimum flow rate and for confirming an empty detection (i.e., determining a false positive empty detection) are described in the context of the flow rate of a return fluid and an empty detection in a return container F4, it will be understood that the methods described herein can be employed on any suitable flow of fluid and container of a disposable circuit 12. In other words, without departing from the scope of the disclosure, the controller 18 can be configured to determine the optimum flow rate for any suitable fluid flowing through disposable circuit 12 and to determine a false positive empty detection of any suitable container of the disposable circuit 12 using the methods described herein.

There are additional aspects to the devices and methods described herein including, without limitation the following aspects.

Aspect 1. A blood processing system including a reusable separation device, wherein the separation device includes a separator and a weight scale; a disposable fluid circuit including a separation chamber and a fluid container, wherein the disposable fluid circuit is configured to be associated with the reusable separation device and the fluid container is configured to be associated with the weight scale; and wherein the reusable separation device includes a controller configured to execute a blood processing procedure and to determine an optimum flow rate for flowing a fluid from the fluid container.

Aspect 2. The system of Aspect 1, wherein the controller is further configured to detect a false positive reading that the fluid container is empty.

Aspect 3. The system of Aspect 2, wherein the controller is configured to detect a false positive reading by pausing the blood processing procedure, recording a first weight of the fluid container after pausing the blood processing procedure, recording a second weight of the fluid container after a period of time after pausing the blood processing procedure, and comparing the second weight to the first weight.

Aspect 4. The system of Aspect 3, wherein the controller is configured to resume the blood processing procedure if the second weight is lower than the first weight by an amount more than a predetermined threshold.

Aspect 5. The system of Aspect 2, wherein the controller is configured to detect a false positive reading by recording a first weight of the fluid container, pumping fluids within the disposable circuit in a reverse direction from an original direction, recording a second weight of the fluid container after a period of time, and comparing the second weight to the first weight.

Aspect 6. The system of Aspect 5, wherein the controller is configured to resume the blood processing procedure if the second weight is greater than the first weight.

Aspect 7. The system of any one of Aspects 5-6, wherein the controller is configured to resume the blood processing procedure if the difference between the second weight and the first weight is greater than a predetermined threshold value.

Aspect 8. The system of Aspect 2, wherein the controller is configured to detect a false positive reading by recording a first weight of the fluid container, isolating a fluid pathway used to fill the fluid container after recording the first weight, pumping a predetermined volume of fluid towards the fluid container via an alternative pathway after isolating the fluid pathway, recording a second weight of the fluid container, and comparing the second weight to the first weight.

Aspect 9. The system of Aspect 8, wherein the controller is configured to resume the blood processing procedure if the second weight is greater than the first weight.

Aspect 10. The system of Aspect 9, wherein the controller is configured to resume the blood processing procedure if the difference between the second weight and the first weight exceeds a predetermined threshold value.

Aspect 11. The system of any one of Aspects 9-10, wherein the controller is configured to resume the blood processing procedure if the difference between the second weight and the first weight exceeds a predetermined threshold percentage of a weight of the predetermined volume of fluid pumped towards the fluid container.

Aspect 12. The system of any one of Aspects 2-11, wherein the controller is configured to determine a fluid hematocrit of the fluid and to record blood processing system events, and wherein the controller is configured to determine the optimum flow rate for flowing the fluid from the fluid container based on the determined fluid hematocrit and recorded blood processing system events.

Aspect 13. The system of Aspect 12, wherein the blood processing events include air purges and occlusions in the disposable fluid circuit.

Aspect 14. The system of any one of Aspects 12-13, wherein the controller is configured to determine the fluid hematocrit based on predetermined hematocrit ranges of components forming the fluid and the recorded blood processing system events.

Aspect 15. The system of any one of Aspects 12-14, wherein the controller is configured to compare the determined fluid hematocrit with predetermined hematocrit ranges stored in the controller, wherein each predetermined hematocrit range is associated with a fluid flow rate, and wherein the controller is configured to flow the fluid from the fluid container at the flow rate associated with the predetermined range within which the fluid hematocrit falls.

Aspect 16. The system of any one of Aspects 2-11, wherein the controller is configured to flow the fluid from the fluid container at a flow rate up to a predetermined limit flow rate.

Aspect 17. The system of Aspect 16, wherein the controller is configured to receive the predetermined limit flow rate from an operator.

Aspect 18. The system of any one of Aspects 2-17, wherein the controller is configured to adjust the flow rate for flowing the fluid from the fluid container after detecting a false positive reading.

Aspect 19. The system of Aspect 18, wherein the flow rate for flowing the fluid from the fluid container is decreased.

Aspect 20. The system of any one of Aspects 1-19, wherein the fluid container is a return fluid container and the fluid is a return fluid.

Aspect 21. A blood processing device including a separator configured to be associated with a separation chamber of a disposable fluid circuit; a plurality of pumps configured to pump fluids throughout the disposable fluid circuit; a weight scale configured to be associated with a fluid container of the disposable fluid circuit; and a controller configured to execute a blood processing procedure and to determine an optimum flow rate for flowing a fluid from the fluid container.

Aspect 22. The device of Aspect 21, wherein the controller is further configured to detect a false positive reading that the fluid container is empty.

Aspect 23. The device of Aspect 22, wherein the controller is configured to detect a false positive reading by pausing the blood processing procedure, recording a first weight of the fluid container after pausing the blood processing procedure, recording a second weight of the fluid container after a period of time after pausing the blood processing procedure, and comparing the second weight to the first weight.

Aspect 24. The device of Aspect 23, wherein the controller is configured to resume the blood processing procedure if the second weight is lower than the first weight by an amount more than a predetermined threshold.

Aspect 25. The device of Aspect 22, wherein the controller is configured to detect a false positive reading by recording a first weight of the fluid container, pumping fluids within the disposable circuit in a reverse direction from an original direction, recording a second weight of the fluid container after a period of time, and comparing the second weight to the first weight.

Aspect 26. The device of Aspect 25, wherein the controller is configured to resume the blood processing procedure if the second weight is greater than the first weight.

Aspect 27. The device of any one of Aspects 25-26, wherein the controller is configured to resume the blood processing procedure if the difference between the second weight and the first weight is greater than a predetermined threshold value.

Aspect 28. The device of Aspect 22, wherein the controller is configured to detect a false positive reading by recording a first weight of the fluid container, isolating a fluid pathway used to fill the fluid container after recording the first weight, pumping a predetermined volume of fluid towards the fluid container via an alternative pathway after isolating the fluid pathway, recording a second weight of the fluid container, and comparing the second weight to the first weight.

Aspect 29. The device of Aspect 28, wherein the controller is configured to resume the blood processing procedure if the second weight is greater than the first weight.

Aspect 30. The device of Aspect 29, wherein the controller is configured to resume the blood processing procedure if the difference between the second weight and the first weight exceeds a predetermined threshold value.

Aspect 31. The device of any one of Aspects 29-30, wherein the controller is configured to resume the blood processing procedure if the difference between the second weight and the first weight exceeds a predetermined threshold percentage of a weight of the predetermined volume of fluid pumped towards the fluid container.

Aspect 32. The device of any one of Aspects 22-31, wherein the controller is configured to determine a fluid hematocrit of the fluid and to record blood processing system events, and wherein the controller is configured to determine the optimum flow rate for flowing the fluid from the fluid container based on the determined fluid hematocrit and recorded blood processing system events.

Aspect 33. The device of Aspect 32, wherein the blood processing events include air purges and occlusions in the disposable fluid circuit.

Aspect 34. The device of any one of Aspects 31-33, wherein the controller is configured to determine the fluid hematocrit based on predetermined hematocrit ranges of components forming the fluid and the recorded blood processing system events.

Aspect 35. The device of any one of Aspects 31-34, wherein the controller is configured to compare the determined fluid hematocrit with predetermined hematocrit ranges stored in the controller, wherein each predetermined hematocrit range is associated with a fluid flow rate, and wherein the controller is configured to flow the fluid from the fluid container at the flow rate associated with the predetermined range within which the fluid hematocrit falls.

Aspect 36. The device of any one of Aspects 22-31, wherein the controller is configured to flow the fluid from the fluid container at a flow rate up to a predetermined limit flow rate.

Aspect 37. The device of Aspect 36, wherein the controller is configured to receive the predetermined limit flow rate from an operator.

Aspect 38. The device of any one of Aspects 22-37, wherein the controller is configured to adjust the flow rate for flowing the fluid from the fluid container after detecting a false positive reading.

Aspect 39. The device of Aspect 38, wherein the flow rate for flowing the fluid from the fluid container is decreased.

Aspect 40. The device of any one of Aspects 21-39, wherein the fluid container is a return fluid container and the fluid is a return fluid.

Aspect 41. A method for processing blood including separating whole blood in a blood processing system configured to execute a blood processing procedure, the system including a reusable separation device, wherein the separation device includes a separator and a weight scale; a disposable fluid circuit including a separation chamber and a fluid container, wherein the disposable fluid circuit is configured to be associated with the reusable separation device and the fluid container is configured to be associated with the weight scale; and wherein the reusable separation device includes a controller configured to execute the blood processing procedure; and determining an optimum flow rate for flowing a fluid from the fluid container.

Aspect 42. The method of Aspect 41, including detecting a false positive reading that the fluid container is empty.

Aspect 43. The method of Aspect 42, wherein detecting a false positive reading includes pausing the blood processing procedure, recording a first weight of the fluid container after pausing the blood processing procedure, recording a second weight of the fluid container after a period of time after pausing the blood processing procedure, and comparing the second weight to the first weight.

Aspect 44. The method of Aspect 43, including resuming the blood processing procedure if the second recorded weight is lower than the first recorded weight above a predetermined threshold amount.

Aspect 45. The method of Aspect 42, wherein detecting a false positive reading includes recording a first weight of the fluid container, pumping fluids within the disposable circuit in a reverse direction from an original direction, recording a second weight of the fluid container after a period of time, and comparing the second weight to the first weight.

Aspect 46. The method of Aspect 45, including resuming the blood processing procedure if the second weight is greater than the first weight.

Aspect 47. The method of any one of Aspect 45-46, including resuming the blood processing procedure if the difference between the second weight and the first weight is greater than a predetermined threshold value.

Aspect 48. The method of Aspect 42, wherein detecting a false positive reading includes recording a first weight of the fluid container, isolating a fluid pathway used to fill the fluid container after recording the first weight, pumping a predetermined volume of fluid towards the fluid container via an alternative pathway after isolating the fluid pathway, recording a second weight of the fluid container after pumping the predetermined volume of fluid, and comparing the second weight to the first weight.

Aspect 49. The method of Aspect 48, including resuming the blood processing procedure if the second weight is greater than the first weight.

Aspect 50. The method of any one of Aspects 48-49, including resuming the blood processing procedure if the difference between the second weight and the first weight exceeds a predetermined threshold value.

Aspect 51. The method of any one of Aspects 48-50, including resuming the blood processing procedure if the difference between the second weight and the first weight exceeds a predetermined threshold percentage of a weight of the predetermined volume of fluid pumped towards the fluid container.

Aspect 52. The method of any one of Aspects 42-51, wherein determining an optimum flow rate for flowing the fluid from the fluid container includes determining a fluid hematocrit of the fluid and recording blood processing system events, and wherein determining the optimum flow rate is based on the determined fluid hematocrit and recorded blood processing system events.

Aspect 53. The method of Aspect 52, wherein the blood processing system events include air purges and occlusions in the disposable fluid circuit.

Aspect 54. The method of any one of Aspects 52-53, wherein determining the fluid hematocrit is based on predetermined hematocrit ranges of components forming the fluid and the recorded blood processing system events.

Aspect 55. The method of any one of Aspects 52-54, wherein determining an optimum flow rate for flowing the fluid from the fluid container includes comparing the determined fluid hematocrit with predetermined hematocrit ranges stored in the controller, wherein each predetermined hematocrit range is associated with a fluid flow rate, and flowing the fluid from the fluid container at the flow rate associated with the predetermined range within which the determined fluid hematocrit falls.

Aspect 56. The method of any one of Aspects 42-47, wherein determining an optimum flow rate for flowing a fluid from the fluid container includes flowing the fluid at a flow rate up to a predetermined limit flow rate.

Aspect 57. The method of Aspect 56, wherein the controller is configured to receive the predetermined limit flow rate from an operator.

Aspect 58. The method of any one of Aspects 42-57, wherein determining an optimum flow rate for flowing the fluid from the fluid container includes adjusting the flow rate after detecting a false positive reading.

Aspect 59. The method of Aspect 58, wherein adjusting the flow rate includes decreasing the flow rate.

Aspect 60. The method of any one of Aspects 42-59, wherein the controller is configured to determine an optimum flow rate for flowing the fluid from the fluid container and to detect a false positive reading that the fluid container is empty.

Aspect 61. The method of any one of Aspects 41-60, wherein the container is a return fluid container and the fluid is a return fluid to be reinfused to a subject.
It will be understood that the embodiments and examples described above are illustrative of some of the applications or principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that the claims may be directed to the features thereof, including as combinations of features that are individually disclosed or claimed herein.

## Claims

1. A blood processing system comprising:
a reusable separation device, wherein the separation device includes a separator and a weight scale;
a disposable fluid circuit including a separation chamber and a fluid container, wherein the disposable fluid circuit is configured to be associated with the reusable separation device and the fluid container is configured to be associated with the weight scale; and
wherein the reusable separation device includes a controller configured to execute a blood processing procedure and to determine an optimum flow rate for pumping a fluid from the fluid container.

2. The system of Claim 1, wherein the controller is further configured to detect a false positive reading that the fluid container is empty.

3. The system of Claim 2, wherein the controller is configured to detect a false positive reading by pausing the blood processing procedure, recording a first weight of the fluid container after pausing the blood processing procedure, recording a second weight of the fluid container after a period of time after pausing the blood processing procedure, and comparing the second weight to the first weight.

4. The system of Claim 3, wherein the controller is configured to resume the blood processing procedure if the second weight is lower than the first weight by an amount more than a predetermined threshold.

5. The system of Claim 2, wherein the controller is configured to detect a false positive reading by recording a first weight of the fluid container, pumping fluids within the disposable circuit in a reverse direction from an original direction, recording a second weight of the fluid container after a period of time, and comparing the second weight to the first weight.

6. The system of any one of Claims 5, wherein the controller is configured to resume the blood processing procedure if the difference between the second weight and the first weight is greater than a predetermined threshold value.

7. The system of Claim 2, wherein the controller is configured to detect a false positive reading by recording a first weight of the fluid container, isolating a fluid pathway used to fill the fluid container after recording the first weight, pumping a predetermined volume of fluid towards the fluid container via an alternative pathway after isolating the fluid pathway, recording a second weight of the fluid container after pumping the predetermined volume of fluid, and comparing the second weight to the first weight.

8. The system of Claim 2, wherein the controller is configured to resume the blood processing procedure if the difference between the second weight and the first weight exceeds a predetermined threshold percentage of a weight of the predetermined volume of fluid pumped towards the fluid container.

9. The system of Claim 2, wherein the controller is configured to determine a fluid hematocrit of the fluid and to record blood processing system events, and wherein the controller is configured to determine the optimum flow rate for flowing the fluid from the fluid container based on the determined fluid hematocrit and recorded blood processing system events.

10. The system of Claim 9, wherein the controller is configured to determine the fluid hematocrit based on predetermined hematocrit ranges of components forming the fluid and the recorded blood processing system events.

11. The system of Claim 9, wherein the controller is configured to compare the determined fluid hematocrit with predetermined hematocrit ranges stored in the controller, wherein each predetermined hematocrit range is associated with a fluid flow rate, and wherein the controller is configured to flow the fluid from the fluid container at the flow rate associated with the predetermined range within which the fluid hematocrit falls.

12. The system of Claim 2, wherein the controller is configured to flow the fluid from the fluid container at a return flow rate up to a predetermined limit flow rate.

13. The system of Claim 2, wherein the controller is configured to adjust the flow rate for flowing the fluid from the fluid container after detecting a false positive reading.

14. A blood processing device comprising:
a separator configured to be associated with a separation chamber of a disposable fluid circuit;
a plurality of pumps configured to pump fluids throughout the disposable fluid circuit;
a weight scale configured to be associated with a fluid container of the disposable fluid circuit; and
a controller configured to execute a blood processing procedure and to determine an optimum flow rate for flowing a fluid from the fluid container, and detect a false positive reading that the fluid container is empty.

15. A method for processing blood comprising:
separating whole blood in a blood processing system configured to execute a blood processing procedure,
determining an optimum flow rate for flowing a fluid from a fluid container of a disposable fluid circuit.
recording a first weight of the fluid container after pausing the blood processing procedure, recording a second weight of the fluid container after a period of time; pausing the blood processing procedure, and comparing the second weight to the first weight.
